(19) Europäisches Patentamt
European Patent Office
Office européen des brevets

(11) **EP 3 045 439 B1**

(12) **EUROPÄISCHE PATENTSCHRIFT**

(45) Veröffentlichungstag und Bekanntmachung des Hinweises auf die Patenterteilung:
**08.11.2017  Patentblatt 2017/45**

(51) Int Cl.:
*C07C 2/10* (2006.01)       *C07C 7/04* (2006.01)
*C07C 11/02* (2006.01)       *C07C 11/08* (2006.01)
*C07C 11/107* (2006.01)

(21) Anmeldenummer: **16151490.6**

(22) Anmeldetag: **15.01.2016**

(54) **KOMBINIERTE HERSTELLUNG VON BUTEN UND OCTEN AUS ETHEN**

COMBINED PRODUCTION OF BUTENE AND OCTENE FROM ETHENE

FABRICATION COMBINEE DE BUTENE ET D'OCTENE A PARTIR D'ETHYLENE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.01.2015  EP 15151621**

(43) Veröffentlichungstag der Anmeldung:
**20.07.2016  Patentblatt 2016/29**

(73) Patentinhaber: **Evonik Degussa GmbH**
**45128 Essen (DE)**

(72) Erfinder:
• **Stochniol, Guido**
  **45721 Haltern am See (DE)**
• **Reeker, Helene**
  **44227 Dortmund (DE)**
• **Peitz, Stephan**
  **45739 Oer-Erkenschwick (DE)**
• **Maschmeyer, Dietrich**
  **45657 Recklinghausen (DE)**
• **Schallenberg, Jörg**
  **46286 Dorsten (DE)**
• **Zanthoff, Horst-Werner**
  **45481 Mülheim a.d. Ruhr (DE)**
• **Häger, Harald**
  **59348 Lüdinghausen (DE)**

(56) Entgegenhaltungen:
**JP-A- 2003 326 169    US-A1- 2013 158 321**

**Beschreibung**

Die Erfindung befasst sich mit der kombinierten Herstellung von Buten und Octen aus Ethen.

Kohlenwasserstoffe sind chemische Verbindungen, die ausschließlich aus Kohlenstoff und Wasserstoff bestehen. Alkene (synonym: Olefine) sind Kohlenwasserstoffe, die eine C=C Doppelbindung im Molekül aufweisen. Alkane (synonym: Paraffine) sind dagegen Kohlenwasserstoffe, die nur Einfachbindungen aufweisen. Sie werden deswegen auch als gesättigt bezeichnet.

In der organischen Chemie werden Kohlenwasserstoffe häufig nach der Anzahl ihrer Kohlenstoffatome pro Molekül bezeichnet, indem der jeweiligen Substanzklasse das Präfix $C_n$ vorangestellt wird. Dabei bezeichnet n die jeweilige Anzahl der Kohlenstoffatome in einem Molekül. So sind $C_4$-Olefine zu verstehen als Substanzen der Klasse der Alkene mit vier Kohlenstoffatomen. $C_8$-Olefine weisen dementsprechend acht Kohlenstoffatome pro Molekül auf. Soweit im Folgenden das Präfix $C_{n+}$ verwendet wird, ist von einer Substanzklasse die Rede, die mehr als n Kohlenstoffatome pro Molekül aufweist. Ein $C_{4+}$-Olefin hat demnach mindestens fünf Kohlenstoffatome.

Das einfachste Olefin ist Ethen (Ethylen). Es weist zwei Kohlenstoffatome auf. Ethen stellt eine bedeutsame Grundchemikalie dar und wird daher in großen Mengen hergestellt. Dies geschieht meist durch Dampfspaltung von Naphtha. Darüber hinaus kann es durch Dehydrierung von Ethan gewonnen werden, welches wiederum ein Bestandteil des Erdgases ist. Aufgrund zunehmender Erschließung von unkonventionellen Erdgasquellen und abnehmender Erdöl-Förderung nimmt der Anteil an auf Erdgas basierendem Ethen stetig zu.

Zu den $C_4$- Olefinen gehören die vier isomeren Stoffe 1-Buten, cis-2-Buten, trans-2-Buten und Isobuten. 1-Buten und die beiden 2-Butene gehören dabei zur Gruppe der linearen Butene, während Isobuten ein verzweigtes Olefin darstellt. Die linearen $C_4$-Olefine 1-Buten, cis-2-Buten und trans-2-Buten werden in der Literatur oft als "n-Buten" zusammengefasst. Abhängig von den thermodynamischen Gegebenheiten treten die vier isomeren $C_4$-Olefine meist gemeinsam auf. Deswegen wird hier bei der Verwendung des Begriffs "Buten" zwischen Einzahl und Mehrzahl nicht unterschieden. Sofern hier ohne weitere Angaben von "Buten" die Rede ist, ist ein lineares Alken mit vier Kohlenstoffatomen (bzw. n-Buten) oder ein Gemisch enthaltend unterschiedliche isomere Alkene mit vier Kohlenstoffatomen gemeint.

Einen aktuellen Überblick über die chemischen und physikalischen Eigenschaften der Butene sowie deren technische Aufarbeitung und Verwertung bieten:

F. Geilen, G. Stochniol, S. Peitz and E. Schulte-Koerne: Butenes. Ullmann's Encyclopedia of Industrial Chemistry. (2013)

Butene fallen heute vorwiegend beim Cracken von Erdölfraktionen in einem Steamcracker oder in einem fluidkatalytischen Cracker (FCC) an und werden als Intermediat für die Herstellung vielfältiger Industrie-Chemikalien verwendet.

Unter einem "Hexen" ist im Folgenden ein Olefin mit sechs Kohlenstoffatomen zu verstehen oder ein Gemisch enthaltend mehrere unterschiedliche $C_6$-Olefine. Bei dem Begriff "Hexen" wird deswegen nicht zwischen Singular und Plural unterschieden. Zu den $C_6$- Olefinen gehören nämlich die achtzehn Isomeren 1-Hexen, (E)-2-Hexen, (Z)-2-Hexen, (E)-3-Hexen, (Z)-3-Hexen, 2-Methyl-1-penten, 2-Methyl-2-penten, (R)-3-Methyl-1-penten, (S)-3-Methyl-1-penten, (E)-3-Methyl-2-penten, (Z)-3-Methyl-2-penten, 4-Methyl-1-penten, (E)-4-Methyl-2-penten, (Z)-4-Methyl-2-penten, (3S)-2,3-Dimethyl-1-buten, (3R)-2,3-Dimethyl-1-buten, 2,3-Dimethyl-2-buten und 3,3-Dimethyl-1-buten.

Von industriellem Interesse sind jedoch nur die Substanzen 1-Hexen und 4-Methyl-1-penten, die als Monomer oder Co-Monomer bei der Herstellung von Kunststoffen eingesetzt werden. Zu diesem Zwecke werden sie im Zuge der Oligomerisierung aus Ethen oder aus dem $C_3$-Olefin Propen hergestellt. Die Oligomerisierung wird später eingehend erläutert.

Unter Octen ist ein Olefin mit acht Kohlenstoffatomen zu verstehen oder ein Gemisch enthaltend mehrere unterschiedliche $C_8$-Olefine. Zu den $C_8$- Olefinen gehört eine Vielzahl von Isomeren, deren Aufzählung hier zu weit führt. Ein industriell wichtiger Vertreter der $C_8$-Olefine ist 1-Octen, welches durch Oligomerisierung von Ethen hergestellt wird und als Co-Monomer in Polyethylen eingesetzt wird.

Ein alternativer Weg zur Herstellung von Octen besteht in der Dimerisierung von n-Buten. Das dabei entstehende Gemisch aus Olefinen mit acht Kohlenstoffatomen wird als Dibuten bezeichnet, es ist also ein besonderes Octen im Sinne der hier gebrauchten Terminologie. Die Besonderheit des Dibutens liegt in der Isomerenverteilung, durch die es sich von anderen Octengemischen unterscheidet.

Je nachdem, auf welche Art und Weise sich die einzelnen n-Buten-Moleküle im Zuge der Oligomerisierung verbinden, erhält man ein Oligomerisat mit unterschiedlichem Verzweigungsgrad. Der Verzweigungsgrad wird durch den Isoindex beschrieben, welcher die mittlere Anzahl der Methylgruppen pro $C_8$-Molekül in dem Isomerengemisch angibt. Der Isoindex für Dibuten ist wie folgt definiert:

Isoindex = (Gewichtsanteil Methylheptene + 2* Gewichtsanteil Dimethylhexene) / 100

So tragen n-Octene mit 0, Methylheptene mit 1 und Dimethylhexene mit 2 zum Isoindex eines Produktgemisches aus $C_8$-Olefinen bei. Je niedriger der Isoindex, desto weniger verzweigt sind die Moleküle innerhalb des Gemisches aufgebaut.

Ein geringer Verzweigungsgrad ist immer dann bedeutsam, wenn das Olefingemisch als Ausgangsstoff für die Herstellung von Weichmachern eingesetzt werden soll. Wissenschaftliche Untersuchungen belegen, dass der Verzweigungsgrad von Olefingemischen, die durch Hydroformylierung, Hydrierung und Veresterung zu Weichmachern weiterverarbeitet werden, für die Eigenschaften und Qualität des Weichmachers maßgeblich ist.

Der Isoindex, den ein $C_8$-Olefingemisch erreichen muss, um als Ausgangsstoff für hochwertige Weichmacher dienen zu können, hängt von den jeweiligen Anforderungen der Weichmacherkunden ab und ändert sich mit der Zeit. Gegenwärtig wird meist ein Isoindex kleiner als 1.1 gefordert.

Unter der inzwischen bereits mehrfach erwähnten Oligomerisierung versteht man die Reaktion von Kohlenwasserstoffen mit sich selbst, wobei entsprechend längerkettige Kohlenwasserstoffe entstehen. Olefine mit zwei bis acht Kohlenstoffatomen lassen sich recht gut oligomerisieren.

So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt. Verbinden sich hingegen drei Olefine mit drei Kohlenstoffatomen miteinander (Trimerisierung), entsteht ein Olefin mit neun Kohlenstoffatomen. Werden n-Butene einer Oligomerisierung unterworfen, entstehen dabei im Wesentlichen Olefine mit acht Kohlenstoffatomen (genauer gesagt: Dibuten) und dazu Olefine mit zwölf Kohlenstoffatomen ($C_{12}$-Olefine, "Tributen"), sowie in einem geringeren Umfang Olefine mit mehr als zwölf Kohlenstoffatomen ($C_{12}$+-Olefine).

Ein industriell praktiziertes Verfahren zur Herstellung von Dibuten durch Oligomerisierung von n-Buten ist der OCTOL®-Prozess. Die ausführliche Beschreibung desselben findet sich in der Nicht-Patentliteratur, beispielsweise unter:

B. Scholz: The HÜLS OCTOL Process: Heterogeneously catalyzed dimerization of n-butenes and other olefins. DGMK-Tagung in Karlsruhe, veröffentlicht in Erdöl, Erdgas, Kohle, April 1989, Seiten 21 und 22.

R.H. Friedlander, D.J. Ward, F. Obenaus, F. Nierlich, J. Neumeister: Make plasticizer olefins via n-butene dimerization. Hydrocarbon Processing, February 1986, Seiten 31 bis 33.

F. Nierlich: Oligomerize for better gasoline. Hydrocarbon Processing, February 1992, Seiten 45 bis 46.

Innerhalb der Patentliteratur beschreibt beispielsweise DE102008007081A1 eine auf dem OCTOL®-Prozess beruhende Oligomerisierung. EP1029839A1 befasst sich mit der Fraktionierung der in dem OCTOL®-Prozess entstehenden $C_8$-Olefine.

Der vollständig heterogen katalysierte OCTOL®-Prozess liefert ein Dibuten mit einem geringen Verzweigungsgrad, welches sich hervorragend zur Herstellung von Weichmachern eignet. Heterogen katalysiert bedeutet, dass der Katalysator als Feststoff in dem flüssigen bzw. gasförmigen Reaktionsgemisch vorliegt. Der Katalysator wird somit von den fluiden Reaktanden umspült und verbleibt im Reaktor.

Unter Co-Oligomerisierung versteht man die gleichzeitige Oligomerisierung von mehreren Substraten in einem Reaktionsgefäß. So beschreibt EP2582648B1 die Co-Oligomerisierung von Buten und Octen unter Erhalt von Dodecen ($C_{12}$-Olefin). Wie in jeder Oligomerisierung weiß man bei einer Co-Oligomerisierung nicht so genau, welches Olefin mit welchem reagiert: So kann in dem Beispiel der EP2582648B1 ein Dodecen sowohl aus drei Butenen, als auch aus einem Buten und einem Octen entstehen. Aus chemischer Sicht kann jede Oligomerisierung als eine Co-Oligomerisierung aufgefasst werden. Aus technischer Sicht hingegen liegt eine Co-Oligomerisierung jedoch nur dann vor, wenn mindestens zwei hinsichtlich ihrer Kohlenstoffanzahl unterschiedliche Olefine in einen gemeinsamen Reaktor eingebracht werden. Es kommt bei der Begriffswahl also auf die steuerbare Zuführung der Einsatzstoffe an, nicht auf die tatsächlich stattfindende Reaktion.

WO2005/123884 offenbart die kombinierte Herstellung von 1-Octen und 1-Hexen durch Tetramerisierung und Trimerisierung von Ethylen. Dafür sind in einem gemeinsamen Reaktionsgefäß zwei unterschiedliche homogene Katalysatoren vorgesehen, nämlich ein erster für die Tetramerisierung und ein zweiter für die Trimerisierung. Da die eingesetzten Homogenkatalysatoren im Reaktionsgemisch gelöst sind, müssen sie entweder durch geeignete Verfahren unter Erhalt ihrer katalytischen Aktivität recycliert oder vollständig abgetrennt werden. Das Recyclieren eines homogenes Katalysators ist mit großem technischem Aufwand und erheblichen apparativen Kosten verbunden, was nur bei sehr teuren Katalysatoren sinnvoll erscheint. Das vollständige Abtrennen eines homogenen Oligomerisierungskatalysators erfolgt meist durch das Quenchen mit Wasser oder alkalischen, wässrigen Lösungen. Dies führt zu einem signifikanten Aufkommen an wässrigen, oftmals Chrom-haltigen Salzlösungen, die entsprechend entsorgt werden müssen. Zudem ergeben sich relativ hohe Kosten für den Einsatz frischer Katalysatorlösungen für die Oligomerisierung.

Darüber hinaus erscheint dieses Verfahren auch nicht dazu geeignet, $C_8$-Olefine für den Einsatz als Ausgangsprodukt für Weichmacher herzustellen: Zwar fallen bei der kombinierten Tetra- und Trimerisierung auch bis zu 52 Gew-% $C_8$-Olefine an, jedoch ohne genaue Angaben zu dem Verzweigungsgrad. Das Verfahren ist im Übrigen auch auf die Produktion des Co-Monomers 1-Octen optimiert, ein $C_8$-Olefin, was sich ohnehin kaum zur Weichmacherherstellung eignet. Es ist daher nicht ersichtlich, dass die $C_8$-Alkene einen Iso-Index erreichen, der sie als Ausgangsprodukt zur Weichmacherherstellung qualifiziert. Darüber hinaus wäre der homogen gelöste Katalysator bei dieser Verwendung definitiv abzutrennen, da die anschließende Hydroformylierung ebenfalls homogen katalysiert wird und auf Störungen von eingeschleppten Fremd-Katalysatoren empfindlich reagiert.

Das eben Gesagte gilt auch für das in WO2005/123633 offenbarte Verfahren zur Oligomerisierung von Ethylen, welches in Gegenwart von Cyclohexan durchgeführt wird. Das Cyclohexan dient dabei als Lösemittel und soll die Desaktivierung des eingesetzten Homogenkatalystors bzw. seines Aktivators verringern.

Ähnliches gilt auch für US2013/0066128 A1 betreffend die homogene Oligomerisierung von Ethen in n-Heptan.

Das Problem der Katalysatorabtrennung besteht nicht bei heterogen katalysierten Prozessen, bei denen der Katalysator als Feststoff vorliegt und im Reaktor verbleibt. Ethylen-Oligomerisierung an einem festen Si/Al/Ni-System ist in US8637722B2 beschrieben. Dieser Prozess findet allerdings in der Gasphase statt, was nachteilig bei der Raumausnutzung der Reaktoren ist. Zudem finden die etablierten Prozessschritte der Weiterverarbeitung von Butenen und Octenen in der Flüssigphase statt, sodass dieser Gasphasenprozess mit bestehender Technologie nicht ohne weiteres kompatibel ist. Eine notwenige Verflüssigung der in der Gasphase gewonnenen Butene und Octene erfordert zusätzliche Energie.

Auch der in WO2010/117539A1 gezeigte Gasphasenprozess zur Oligomerisierung von in einem FCC-Gas verdünnten Ethylen an einem zeolithischen Ni-Katalysator lässt sich nicht ohne weiteres in einen etablierten Produktionsstrang zur C4/C8 Verwertung eingliedern.

Eine Mischform zwischen heterogener und homogener Oligomerisierung zeigt US2013/0158321A1. Hier wird Ethen zunächst homogen zu Butenen dimerisiert und diese anschließend durch heterogene Katalyse an einem festen Nickelkontakt in Octene umgesetzt. Beide Reaktionsstufen finden in der flüssigen Phase in Gegenwart von Hexan statt. Der Reaktionsaustrag der ersten Stufe muss mit Base neutralisiert und destillativ von dem Homogenkatalysator (Triethylaluminium) befreit werden. In der industriellen Praxis ist dies sehr aufwendig.

Im Lichte dieses Standes der Technik bestand die Aufgabe der vorliegenden Erfindung darin, ein Verfahren zur kombinierten Herstellung von Buten und Octen aus Ethen anzugeben, welches vornehmlich $C_8$-Olefine mit hoher Linearität (also geringem Verzweigungsgrad, kleinem Isoindex) liefert. Darüber hinaus sollte der Prozess eine hohe Ausbeute von 1-Buten besitzen, da dieses begehrte Isomer sich separat vermarkten lässt. Er sollte vollständig heterogen katalysiert sein, damit keine aufwändige Katalysatorabtrennung betrieben werden muss, um Verunreinigungen nachgelagerter, homogen katalysierter Prozesse zu vermeiden. Schließlich soll der Prozess möglichst in der flüssigen Phase durchgeführt werden können, um mit etablierten Technologien zur Verwertung von Buten und Octen kompatibel zu sein.

Gelöst wird diese Aufgabe durch eine simultane Durchführung zweier Reaktionen, nämlich eine erste Synthese vornehmlich von $C_2$ zu $C_4$ und eine zweite Synthese von $C_2$ und $C_6$ zu $C_8$. Beide Reaktionen werden räumlich getrennt voneinander und dementsprechend in unterschiedlichen Reaktoren oder zumindest in unterschiedlichen, räumlich getrennten Bereichen eines Reaktors betrieben.

Eine besondere Ausführungsform der Erfindung besteht darin, dass beide Synthesen in Gegenwart eines inerten Lösemittels durchgeführt werden. Mit Hilfe des Lösemittels lässt sich nämlich die Selektivität der Reaktionen in Richtung Buten bzw. Octen beeinflussen. Insbesondere in der zweiten Synthese kann durch gezielte Einstellung der Konzentration von Ethen in dem Lösemittel bzw. dem Hexen die Reaktion aus $C_2+C_6$ gegenüber der Reaktion von $C_2+C_2$ begünstigt werden.

Das eingesetzte inerte Lösemittel muss zwei Voraussetzungen erfüllen:

Zunächst muss es inert sein. Dies bedeutet, dass es bei den im Verfahren herrschenden Bedingungen (Druck/Temperatur in den Reaktionen und Kolonnen) an keinen chemischen Reaktionen teilnimmt. Insbesondere sollte es nicht mit Ethen reagieren. Das Lösemittel wird im Prozess also nicht verbraucht. Natürlich wird sich nicht ausschließen lassen, dass das Lösemittel nicht doch in irgendeiner Form irgendwo reagiert. Jedoch sollten diese Reaktionen im Vergleich zu den gewünschten Reaktionen so langsam verlaufen, dass sie nicht signifikant sind. Die langsame Reaktion darf sich allenfalls als Altern des Lösemittels bemerkbar machen.

Zum zweiten sollte das Lösemittel einen Siedepunkt aufweisen, der zwischen den Siedepunkten der Butene und denen der Octene angesiedelt ist. Es sollte also höher sieden als das höchst siedende Buten im Prozess; aber niedriger, als das im Prozess anwesende Octen mit dem niedrigsten Siedepunkt. Sofern das Lösemittel keinen singulären Siedepunkt aufweist, sondern einen Siedebereich (etwa, weil es sich bei dem Lösemittel um ein Gemisch und nicht um einen Reinstoff handelt) sollte der Siedebereich des Lösemittels zwischen den Butenen und den Octenen liegen. Die hier verglichenen Siedepunkte verstehen sich als Siedetemperaturen bei denselben Drücken.

Ein Lösemittel, welches beide Voraussetzungen (Siedepunktlage, inertes Verhalten) erfüllt, ist beispielsweise das $C_6$-Alkan n-Hexan.

Der Vorteil der Verwendung eines Lösemittel mit dieser Siedepunktlage besteht darin, dass es sich einfach über den Kolonnensumpf der Buten-Kolonne abtrennen lässt, wohingegen etwa ein leichteres Lösemittel eine Abtrennung über Kopf erfordert, was mit einem größeren Energieaufwand einhergeht. Ein Teil des Lösemittels bildet schließlich das Hexen, was im Prozess als Nebenprodukt gebildet wird und deswegen nicht gesondert beschafft werden muss. Da die Siedelage des Lösemittels im Wesentlichen dem des Nebenprodukts Hexen entspricht, kann das Nebenprodukt gemeinsam mit dem Lösemittel als Mittelsieder aus dem ersten Reaktionsgemisch abgetrennt und in die zweite Reaktion überführt werden.

Die Verwendung von dem inerten Lösemittel verhindert jedoch nicht, dass in der ersten Synthese neben dem gewünschten Buten auch noch Hexen, Octen und höhere Oligomere gebildet werden.

Eine weitere Besonderheit des erfindungsgemäßen Verfahrens besteht darin, dass das in der ersten Synthese auf dem Wege der $C_2$-Trimerisierung gebildete Hexen als Edukt für die zweite Synthese verwendet wird. Dort wird es mit Ethen zu Octen umgesetzt, dem zweiten Zielprodukt. Das eigentlich unerwünschte Nebenprodukt der ersten Synthese (Hexen) wird somit zur Bildung des Zielprodukts Octen weiterverwertet. Zudem wird Hexen laufend im Kreis gefahren und als Lösungsmittel für Ethen genutzt. Dies ermöglicht es nämlich, beide Synthesen in der Flüssigphase durchzuführen und heterogen zu katalysieren. Dies sind weitere wesentliche Aspekte des hier vorgestellten Produktionsprozesses.

Konkret ist ein Verfahren zur kombinierten Herstellung von Buten und Octen aus Ethen mit den Schritten a) bis i) Gegenstand der Erfindung:

a) Bereitstellen eines Lösemittels, dessen Siedepunkt bzw. Siedebereich oberhalb der Siedepunkte der Butene und unterhalb der Siedepunkte der Octene liegt; und bei welchem es sich um ein inertes Lösemittel handelt oder um Hexen allein oder um Hexen gemischt mit Pentan oder Hexan oder Heptan oder um eine Mischung aus Pentan, Hexan, Heptan;
b) Bereitstellen eines ersten Einsatzgemisches enthaltend zumindest das Lösemittel und darin gelöstes Ethen;
c) Bereitstellen eines zweiten Einsatzgemisches enthaltend zumindest Hexen, das Lösemittel, sowie im Lösemittel und/oder im Hexen gelöstes Ethen;
d) Überführen des ersten Einsatzgemisches in eine erste Synthese und des zweiten Einsatzgemisches in eine zweite Synthese, wobei erste und zweite Synthese räumlich voneinander getrennt sind;
e) in der ersten Synthese Oligomerisieren zumindest eines Teils des im ersten Einsatzgemisch enthaltenden Ethens in Gegenwart eines ersten heterogenen Katalysators und in Gegenwart des Lösemittels unter Erhalt eines ersten Reaktionsgemisches umfassend zumindest das Lösemittel, Buten und Hexen;
f) Abtrennen einer Buten enthaltenden Leichtsiederfraktion aus dem ersten Reaktionsgemisch bzw. aus einem auf dem ersten Reaktionsgemisch basierenden Stoffstrom;
g) Abtrennen einer Hexen und das Lösemittel enthaltenden Mittelsiederfraktion aus dem ersten Reaktionsgemisch bzw. aus einem auf dem ersten Reaktionsgemisch basierenden Stoffstrom;
h) Verwenden zumindest eines Teils der Mittelsiederfraktion im Zuge der Bereitstellung des zweiten Einsatzgemischs;
i) in der zweiten Synthese Umsetzen zumindest eines Teils des im zweiten Einsatzgemisch enthaltenden Ethens mit zumindest einem Teil des im zweiten Einsatzgemisch enthaltenden Hexens in Gegenwart eines zweiten heterogenen Katalysators und in Gegenwart des Lösemittels unter Erhalt eines zweiten Reaktionsgemisches umfassend zumindest Octen und das Lösemittel.

Da Ethen sehr reaktiv ist, kann es in der ersten und zweiten Synthese vollständig umgesetzt werden. Allerdings empfiehlt es sich, die erste und/oder die zweite Reaktion vor dem vollständigen Umsatz des Ethens abzubrechen, sodass im ersten Reaktionsgemisch bzw. im zweiten Reaktionsgemisch unverbrauchtes Ethen zurück bleibt. Das unverbrauchte Ethen ist aus dem jeweiligen Reaktionsgemisch abzutrennen und zur Bereitstellung des ersten und/oder zweiten Einsatzgemisches wiederzuverwerten.

Der Hintergrund für den unvollständigen Umsatz des Ethens besteht darin, dass der Katalysator rasch beginnt, die 1-Alkene zu isomerisieren, sobald das Ethen bis zu einem gewissen Grade verbraucht wurde. Die Isomerisierung läuft teilweise auch schon vorher ab, wobei dann aber die aus Ethen gebildeten Butene mit dem Ethen um die katalytisch aktiven Zentren konkurrieren. Also wird durch einen Abbruch der Reaktion vor vollständigem Ethen-Verbrauch ein höherer 1-Buten-Gehalt erzielt.

Erfindungsgemäß wird die erste Synthese auf die Produktion von 1-Buten optimiert. Zusätzlich wird unvermeidbar in der zur Produktion von Octen bestimmten, zweiten Synthese $C_2$ zu $C_4$ dimerisiert. Es empfiehlt sich, das als Nebenprodukt in der zweiten Synthese gebildete, und damit im zweiten Reaktionsgemisch enthaltende Buten gemeinsam mit dem im ersten Reaktionsgemisch enthaltenden Buten aufzuarbeiten. Durch diese Maßnahme werden Destillationskolonnen eingespart. Zwar müssen die gemeinsam genutzten Kolonnen größer dimensioniert werden, was die Investitionskosten steigert, aber der Betrieb ist dann günstiger, da Wartungs- und Energiekosten geringer ausfallen.

Das Abtrennen von im ersten Reaktionsgemisch enthaltenden Buten und dem Lösemittel erfolgt konventionell mit einer Anordnung von Destillationskolonnen, die nacheinander die Fraktionen hinsichtlich ihrer Anzahl an Kohlenstoffatomen zerlegen. Alternativ kann die destillative Aufarbeitung mit Seitenabzugskolonnen erfolgen. Die destillative Abtrennung erfolgt unmittelbar aus dem ersten Reaktionsgemisch oder aus einem darauf basierenden Stoffstrom. Bei letzterem kann es sich um ein Gemisch aus einem weiteren prozessinternen Stoffstrom und dem ersten Reaktionsgemisch handeln oder um einen Rest des ersten Reaktionsgemisches, nachdem aus diesem bereits eine Fraktion abgetrennt wurde, wie etwa $C_2$.

Es wird nicht möglich sein, das Hexen in der zweiten Synthese vollständig umzusetzen. Grund dafür ist, dass das Hexen nicht nur als Edukt sondern auch teilweise als Lösemittel für Ethen genutzt wird, um es in der flüssigen Phase zu halten. Es besteht daher ein Überangebot an Hexen in der zweiten Synthese, was dazu führt, dass sich nicht umgesetztes Hexen im zweiten Reaktionsgemisch findet. Dem erfindungsgemäßen Aspekt der Kreislauffahrweise von Lösemittel mit Hexen folgend sollte es aus dem zweiten Reaktionsgemisch abgetrennt und zumindest teilweise in die zweite Synthese zurückgeführt werden. Die Abtrennung des Hexens und des Lösemittels aus dem zweiten Reaktionsgemisch kann dadurch erfolgen, dass dieses mit dem ersten Reaktionsgemisch verschnitten wird, sodass die Abtrennung der Fraktion aus von Hexen und Lösemittel aus dem zweiten Reaktionsgemisch zusammen mit der Abtrennung von Hexen und Lösemittel aus dem ersten Reaktionsgemisch erfolgt, wodurch wieder der Synergieeffekt der Kolonneneinsparung erzielt wird.

Auf der einen Seite ist es wichtig, dass das Ethen möglichst weitestgehend in dem Lösemittel gelöst vorliegt, da eine Gasphase aus ungelöstem Ethen die Prozessintensität in den beiden heterogen katalysierten Synthesen schmälert: Ethylen-Gasblasen im flüssigen Lösemittel erhöhen das Volumen des Einsatzgemisches, was wiederum ein größeres Katalysator- und Reaktorvolumen erforderlich macht. Auch wird die Strömungsdynamik im Katalysatorbett durch ungelöstes Ethen weniger beherrschbar. Zudem kann ein direkter Kontakt des unverdünnten gasförmigen Ethens mit dem Katalysator zu verstärkt auftretender stark exothermer Polymerisation führen; deshalb sollte der Katalysator nach Möglichkeit stets mit Flüssigkeit benetzt sein.

Auf der anderen Seite können eine hohe Lineargeschwindigkeit in Verbindung mit einer Sprudelphase mit hinreichend hoher Flüssigkeitsbelastung (puls flow regime) dafür sorgen, dass durch ein Arbeiten im Bereich der turbulenten Flüssigkeitsströmung die Flüssigphase schneller nachgesättigt wird, wie man aus Erfahrungen mit Hydrierungen weiß (z. B. EP 0319208A1).

Deshalb besteht alternativ die Möglichkeit die Reaktion in der Sprudelphase mit hinreichend hoher Flüssigkeitsbelastung durchzuführen, das heißt am Eingang des Reaktors mit einem Teil des Ethens in der Gasphase, also als Ethen-Gasblasen im flüssigen $C_5$- bis $C_7$-Lösemittel, welche zur schnellen Nachsättigung der Flüssigphase im weiteren Reaktionsverlauf dienen.

Das erste Zielprodukt des erfindungsgemäßen Prozesses ist 1-Buten. Es hat unter allen Butenen die beste Reaktivität und Linearität und ist daher ein begehrter Synthesebaustein, der möglichst isomerenrein gewonnen werden sollte. Dies ist möglich durch eine Destillation, in welcher im abgetrennten Buten enthaltendes 1-Buten gewonnen wird, indem es von im abgetrennten Buten enthaltenden 2-Buten destillativ getrennt wird. Die destillative Trennung der Isomeren ist aufgrund der Siedepunktlage wirtschaftlich. Dabei geht 1-Buten über Kopf, während cis-2-Buten und trans-2-Buten im Sumpf verbleiben.

Getrieben wird diese Trennung durch einen kontinuierlichen Abzug von 2-Buten aus dem Sumpf der Destillationskolonne. Diese beiden dort erhaltenen, weniger attraktiven 2-Butene lassen sich durch eine Isomerisierung aufwerten, in welcher im abgetrennten Buten enthaltendes 2-Buten zumindest teilweise zu 1-Buten isomerisiert wird; unter Erhalt eines Isomerisats, welches in die Destillation, in der 1-Buten gewonnen wird, zurückgeführt wird. Isomerisierung bedeutet in diesem Zusammenhang, dass das thermodynamische Gleichgewicht in einem Gemisch aus 1-Buten, cis-2-Buten und trans-2-Buten zu Gunsten von 1-Buten verschoben wird. Die Isomerisierung von 2-Buten zu 1-Buten ist beispielsweise in EP0718036A1 beschrieben. Die Isomerisierung vermag also die Ausbeute an 1-Buten zu steigern. Zusätzlich erlaubt sie eine Energieeinsparung, da die Destillation, an deren Kopf 1-Buten gewonnen wird, weniger scharf gefahren werden muss, weil 1-Buten im Sumpf verbleiben darf. Die Isomerisierung braucht im Übrigen nicht so groß dimensioniert zu werden, wenn am Sumpf der Destillation, in der 1-Buten gewonnen wird, 2-Buten aus dem Prozess ausgeschleust wird.

Eine zweite Möglichkeit zur Verwertung von 2-Buten aus dem Sumpf der Kolonne zur Abtrennung von 1-Buten besteht in der Durchführung einer oxidativen Dehydrierung zu Butadien. Die Umsetzung zu Butadien muss nicht vollständig erfolgen. Butadien, genauer gesagt 1,3-Butadien, wird in großen Mengen zur Herstellung von Synthesekautschuk verwendet und lässt sich deswegen gut vermarkten. Vor der oxidativen Dehydrierung kann noch eine Isomerisierung von 2-Buten zu 1-Buten durchgeführt werden. Die benötigte Technologie zur Herstellung von Butadien durch oxidative Dehydrierung von n-Buten nach vorhergehender Isomerisierung ist ausführlich in der zum Anmeldezeitpunkt noch unveröffentlichten deutschen Patentanmeldung 102013226370.8 geschildert.

Schließlich kann das 2-Buten vom Sumpf der zur Abtrennung von 1-Buten bestimmten Kolonne noch zumindest teilweise zu Octen umgesetzt werden, unter Erhalt eines dritten Reaktionsgemisches, welches gemeinsam mit dem ersten Reaktionsgemisch aufgearbeitet werden kann. Bei der Umsetzung selbst handelt es sich um eine Oligomerisierung, die entsprechend des OCTOL®-Prozesses durchgeführt werden kann, aber nicht muss. Die Durchführung entsprechend des OCTOL®-Prozesses ist aber bevorzugt, da dieser hochwertiges Dibuten liefert. Die Aufarbeitung des so gewonnen Oligomerisats aus der dritten Synthese erfolgt zusammen mit dem Oligomerisat der $C_2$-Oligomerisierung, da so Kolonnen eingespart werden.

Die erste Synthese sollte bei einer Temperatur zwischen 20 °C und 150 °C und bei einem Druck zwischen $1*10^5$ Pa und $50*10^5$ Pa erfolgen, wobei die Prozessbedingungen so gewählt sind, dass das Lösemittel flüssig vorliegt.

Bevorzugt beträgt der Anteil des Ethens an dem ersten Einsatzgemisch zwischen 1 Gew.-% und 50 Gew.-%. Im Interesse der Prozessintensität sollte dabei der Anteil des Ethens an dem ersten Einsatzgemisch und die Reaktionsbedingungen der ersten Synthese so aufeinander abgestimmt werden, dass in der ersten Synthese das Lösemittel in der flüssigen Phase vorliegt.

Das Ethen kann dann entweder vollständig gelöst in dem Lösemittel sein, sodass die Reaktion vollständig in der flüssigen Phase stattfindet. Die Ethen-Konzentration in dem Lösemittel und die Reaktionsbedingungen sind dementsprechend so zu wählen, dass das Ethen stets gelöst bleibt.

Alternativ kann die Reaktion auch in der Sprudelphase durchgeführt werden. Das bedeutet, dass das Lösemittel zwar flüssig vorliegt mit darin gelöstem Ethen, dass aber ein Teil des Ethens auch in der Gasphase vorliegt und damit eine Gas/flüssig-Reaktion durchgeführt wird. Die Ethen-Konzentration in dem Lösemittel und die Reaktionsbedingungen sind dementsprechend so zu wählen, dass das Ethen teilweise gelöst ist und teilweise in der Gasphase vorliegt.

Die erste Synthese wird vorzugsweise mit einem Umsatz zwischen 50 % und 100 %, einer Selektivität zu $C_4$ von 50 % bis 95 % und einer Selektivität zu $C_8$ von 0 % bis 20 % gefahren.

Auch in der zweiten Synthese sind die Reaktionsbedingungen so zu wählen, dass das das Hexen sowie etwaiges weiteres Lösemittel in der flüssigen Phase vorliegt. Der bevorzugte Anteil an Ethen im zweiten Einsatzgemisch liegt zwischen 0,1 Gew.-% und 30 Gew.-%; die zweite Synthese sollte bei einer Temperatur zwischen 20°C bis 150 °C und bei einem Druck zwischen $1*10^5$ Pa und $50*10^5$ Pa erfolgen. Die zweite Synthese wird vorzugsweise mit einem Umsatz zwischen 90 % und 100 %, einer Selektivität zu $C_4$ von 0 % bis 70 % und einer Selektivität zu $C_8$ von 20 bis 80 % gefahren.

Grundsätzlich sollte das zweite Einsatzgemisch mehr Gewichtsanteil Hexen als Ethen enthalten, um ein Überangebot von Hexen zu schaffen.

Bevorzugt beträgt der Anteil des Ethens an dem zweiten Einsatzgemisch weniger als 30 Gew.-%, ganz besonders bevorzugt weniger als 20 Gew.-%. Durch einen Überschuss an $C_6$ gegenüber $C_2$ wird nämlich die Reaktion von $C_6$ mit $C_2$ gegenüber $C_2$ mit $C_2$ begünstigt.

Um beide Reaktionen in der flüssigen Phase bzw. in der Sprudelphase durchführen zu können, werden beide Reaktionen in einem bei Reaktionsbedingungen flüssigen Lösemittel durchgeführt.

Bei dem Lösemittel handelt es sich vorzugsweise um mindestens einen Kohlenwasserstoff mit fünf, sechs oder sieben Kohlenstoffatomen. Kohlenwasserstoffe mit mehr als sieben Kohlenstoffatomen können nicht verwendet werden, da diese im Kolonnensumpf verbleiben und gesondert von dem Wertprodukt Octen abgetrennt werden müssen. Kohlenwasserstoffe mit weniger als fünf Kohlenstoffatomen sind als Lösemittel ebenfalls weniger geeignet, da diese in der Destillation über Kopf gehen und deswegen einen größeren Energieaufwand erfordern. Mithin ist ein Lösemittel zu verwenden, dessen Siedepunktlage zwischen dem des ersten Zielprodukts 1-Buten und dem des zweiten Zielprodukts 1-Octen liegt. Dies sind die Kohlenwasserstoffe mit fünf, sechs und sieben Kohlenstoffatomen. Es können auch Mischungen unterschiedlicher Kohlenwasserstoffe mit derselben oder unterschiedlicher Anzahl an Kohlenstoffatomen verwendet werden. Dies führt in der Regel dazu, dass das als Lösemittel verwendete Gemisch nicht einen singulären Siedepunkt aufweist, sondern einen Siedebereich, der entsprechend der Maßgabe zwischen den beiden Zielprodukten liegt. Der Vergleich der Siedepunkte versteht sich als Vergleich der Siedetemperaturen bei gleichem Druck.

Vorzugsweise werden die entsprechenden C5- bis C7- Alkane als Lösemittel verwendet. Alkane sind nämlich aufgrund ihrer gesättigten Verbindungen weitaus weniger reaktiv als Alkene und verhalten sich deshalb in beiden Reaktionen inert. Da sie sich in der Reaktion nicht verändern, ist es verfahrenstechnisch einfacher, den Kreislauf durch die beiden Reaktionen aufrecht zu erhalten. Als Lösemittel wird deswegen Pentan, Hexan oder Heptan verwendet, einzeln oder miteinander gemischt. Es können auch die zyklischen Alkane Cycopentan, Cyclohexan und Cycloheptan verwendet werden.

Entsprechend werden die beiden Einsatzgemische jeweils so bereitgestellt, dass sie jeweils mindestens ein Alkan mit fünf oder sechs oder sieben Kohlenstoffatomen enthalten.

Neben den $C_5$, $C_6$, $C_7$-Alkanen kann das $C_6$-Olefin Hexen als Lösemittel verwendet werden, da es die gewünschte Siedepunktlage hat. Sofern in Summe über die beiden Reaktionen genauso viel Hexen gebildet wird, wie umgesetzt wird, ist der Kreislauf stabil. In der Gesamtbilanz verhält sich Hexen quasi inert, obwohl es in den Einzelbilanzen um die jeweiligen Reaktionen sehr wohl umgesetzt wird. Sofern die Hexen-Bilanz nicht ausgeglichen ist, kann Hexen von außen zugegeben werden oder nach außen abgeführt werden. In einem solchen Fall empfiehlt es sich einen Pufferspeicher für Hexen anzulegen, in welchen eine Überproduktion von Hexen abgeführt wird und aus welchem im Falle einer Unterproduktion Hexen entnommen wird.

Als Lösemittel kann mithin Hexen allein oder gemischt mit Pentan oder Hexan oder Heptan oder Mischungen dieser $C_5$- bis $C_7$-Alkane verwendet werden. Die Verwendung von Hexen allein als Lösemittel stellt ein Sonderfall der vorliegenden Erfindung dar, in dem ein reaktives Lösemittel verwendet wird. Die $C_5$ und $C_7$ Olefine sind hingegen nicht als Lösemittel geeignet, da sie sich in den Reaktionen nicht inert verhalten und stattdessen unerwünschte Nebenprodukte bilden, die wiederum aufwändig abgetrennt werden müssen.

Als optimales Lösemittel hat sich ein Gemisch aus Hexan und Hexen erwiesen: In Gegenwart von Hexan wird nämlich in der ersten Reaktion weniger Hexen verbraucht, sodass der Lösemittelkreislauf aufrechterhalten werden kann. Pures Hexen ist in zu hohen Konzentrationen so reaktiv, dass es vermehrt mit sich selbst zu $C_{8+}$ umgesetzt wird und damit mehr $C_6$ verbraucht wird als vorgelegt wurde. Das genaue Mengenverhältnis von Hexan zu Hexen ist in Hinblick auf den Umsatz von Hexen und Ethen zu Octen (in der zweiten Reaktion) auszutarieren und hängt im Wesentlichen von dem eingesetzten Katalysator ab.

Ein besonderer Vorteil des hier vorgestellten Verfahrens gegenüber industriell betriebener Octen-Herstellung besteht darin, dass hier ein heterogener Katalysator eingesetzt wird, der im Reaktor verbleibt und nicht im Produkt. Bei dem Katalysator handelt es sich mithin um einen Festkörper, der vorzugsweise als Festbett in dem jeweiligen Reaktor eingebaut ist. Für die Durchführung der ersten und der zweiten Synthese können derselbe oder unterschiedliche Katalysatoren vorgesehen sein. Erster und zweiter Katalysator können also identisch sein, müssen es aber nicht. Beide Katalysatoren sind aber räumlich voneinander getrennt anzuordnen, bestenfalls in unterschiedlichen Reaktionsgefäßen, aber zumindest als unterschiedliche Schichten in demselben Reaktionsgefäß.

Als erster und/oder zweiter heterogener Katalysator eignet sich konkret ein Festkörper, welcher mindestens zwei Komponenten enthält, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus $Al_2O_3$, $SiO_2$, $TiO_2$, $ZrO_2$ ausgewähltes Metalloxid umfasst.

Besonders bevorzugt kann ein Katalysator verwendet werden, der als erste Komponente Nickel und als zweite Komponente Silica umfasst. Ein solcher Katalysator ist in US2581228 offenbart. Mögliche Beispiele zum Layout eines Koppelprozesses, der sowohl die $C_2$-Oligomerisierung als auch die Umsetzung von Ethylen mit Hexen in Gegenwart von Hexan als Lösemittel umfasst, sollen nun anhand der Figuren näher erläutert werden. Hierfür zeigen:

Fig.1: Grundkonzept erfindungsgemäßes Verfahren;

Fig.2: Variante mit Isomerisierung;

Fig.3: Variante mit oxidativer Dehydrierung;

Fig.4: Variante mit $C_4$-Oligomerisierung.

Alle Figuren sind schematisch und zeigen lediglich die wesentlichen Bestandteile einer entsprechenden Anlage zur Durchführung des erfindungsgemäßen Verfahrens.

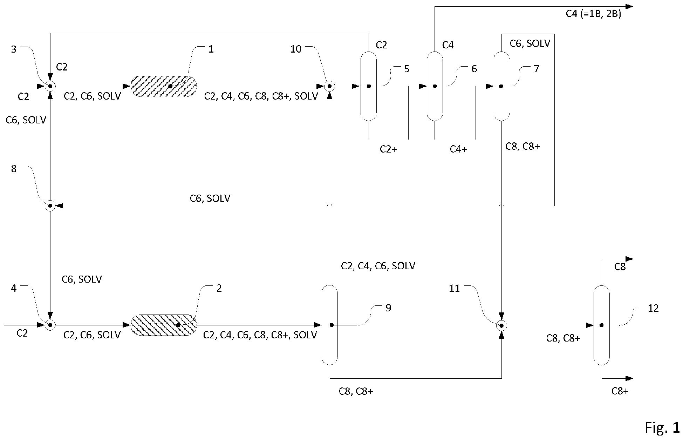

Figur 1 zeigt das Grundprinzip. Es sieht zwei parallel betriebene Synthesen 1, 2 vor, die in räumlich getrennten Reaktoren durchgeführt werden. Bei der ersten Synthese 1 handelt es sich um eine Ethen-Oligomerisierung. Sie dient vornehmlich zur Herstellung von Buten. Die zweite Synthese 2 dient zur Herstellung von Octen aus Ethen und Hexen.

Das für beide Synthesen 1,2 benötigte Ethen C2 stammt aus einer oder mehreren hier nicht dargestellten Quellen. Die Reinheit des flüssig oder gasförmig anströmenden Ethens C2 beträgt mehr als 99,9%. Als Begleitstoffe können weniger als 10 ppm Sauerstoff, weniger als 5 ppm Kohlenmonoxid, weniger als 10 ppm Kohlendioxid und weniger als 1000 ppm anderer Kohlenwasserstoffe auftreten. Eine höhere Reinheit ist nicht erforderlich, da die häufigsten Verunreinigungen inerte Alkane wie Ethan oder Methan sind, welche die Reaktion selbst nicht stören und lediglich bei höheren Anteilen die Siede- und Druckbereiche leicht verändern.

Jeder Synthese 1,2 ist jeweils ein Mischer 3, 4 zugeordnet. Der erste Mischer 3 dient dazu, für die erste Synthese 1 ein erstes Einsatzgemisch C2, C6, SOLV bereitzustellen. Bei dem ersten Einsatzgemisch handelt es sich um ein flüssiges Hexen/Hexan-Gemisch C6, SOLV mit darin vollständig gelöstem Ethen C2. Das Hexen/Hexan-Gemisch C6, SOLV stammt aus einem C6-Rücklauf, das Ethen C2 zum Teil aus der Quelle und zum Teil aus einem Ethen-Rücklauf. Die Zusammensetzung des ersten Einsatzgemisches wird in dem ersten Mischer 3 so eingestellt, dass es bei den Reaktionsbedingungen in der ersten Synthese flüssig ist und das Ethen vollständig in dem Hexan/Hexen-Gemisch gelöst ist.

In der ersten Synthese 1 wird das Ethen in Gegenwart eines ersten heterogenen Katalysators und in Gegenwart des Hexens und des Hexans oligomerisiert. Dabei entstehen Butene C4, Hexene C6, Octene C8 und höhere Olefine C8+. Ein Teil des Ethens C2 wird nicht umgesetzt. Ob Hexen C6 umgesetzt wird, ist unbekannt. Möglich ist dies, aber es lässt sich nicht feststellen, da durch Trimerisierung von Ethen gleichzeitig neues Hexen gebildet wird. Allerdings sieht es die Verfahrensführung vor, dass in der Massenbilanz um die erste Synthese Hexen im Austrag zunimmt. Es wird also in der ersten Synthese 1 netto mehr Hexen gebildet als umgesetzt. Insgesamt umfasst der erste Reaktionsaustrag C2, C4, C6, C8, C8+, SOLV also nicht umgesetztes Ethen, selektiv gebildetes Buten, altes und frisch gebildetes Hexen, gebildetes Octen und gebildete höhere Olefine. Da sich das Hexan SOLV in der Reaktion inert verhält, findet sich dieses auch wieder im ersten Reaktionsgemisch.

Das erste Reaktionsgemisch C2, C4, C6, C8, C8+, SOLV wird mit Hilfe einer Serie aus drei Kolonnen 5, 6, 7 destillativ aufgearbeitet. Die erste Kolonne 5 trennt Ethen C2 über Kopf ab, sodass die Olefine mit mehr als zwei Kohlenstoffatomen C2+ im Sumpf bleiben. Das über Kopf der ersten Kolonne 5 abgezogene Ethen C2 wird als Ethen-Rücklauf in den ersten Mischer 3 zurückgeführt.

Die zweite Kolonne 6 trennt nun über Kopf die aus dem ersten Reaktionsgemisch stammenden Butene C4 als eine Leichtsiederfraktion ab. Die Leichtsiederfraktion enthält im Wesentlichen 1-Buten 1B und cis/trans-2-Buten 2B. Die Olefine mit mehr als vier Kohlenstoffatomen C4+ werden vom Sumpf der zweiten Kolonne 6 in die dritte Kolonne 7 geführt. Dort wird über Kopf Hexen C6 und Hexan SOLV als Mittelsiederfraktion abgetrennt, sodass Octen C8 und die höheren Olefine C8+ im Sumpf verbleiben.

Die an der dritten Kolonne 7 abgetrennte Mittelsiederfraktion aus Hexen C6 und Lösemittel Hexan SOLV wird rezykliert zu einem Teiler 8, der den Mittelsieder-Rücklauf aufteilt auf den ersten Mischer 3 und den zweiten Mischer 4.

Im zweiten Mischer 4 wird in dem rezyklierten Mittelsieder aus Hexen C6 und Hexan SOLV frisches Ethen C2 gelöst, sodass ein zweites Einsatzgemisch C2, C6, SOLV entsteht. Zu dem frischen Ethen kann auch Rücklauf-Ethen von der ersten Kolonne 5 oder von einer hinter der zweiten Synthese 2 angeordneten Ethen-Kolonne zugegeben werden, was aber bei der in Fig. 1 dargestellten Ausführungsform nicht der Fall ist.

Die Zusammensetzung des zweiten Einsatzgemisches C2, C6 wird in dem zweiten Mischer 4 so eingestellt, dass es bei den Reaktionsbedingungen in der zweiten Synthese 2 flüssig ist und das Ethen vollständig im Hexen/Hexan-Gemisch gelöst ist.

In der zweiten Synthese wird nun heterogen katalysiert Ethen C2 und Hexen C6 zu Octen C8 umgesetzt. Durch die Anwesenheit des inerten Hexans als Lösemittel SOLV wird die Reaktion in Richtung Octen optimiert. Dennoch finden in der zweiten Synthese 2 noch Nebenreaktionen statt, denn es werden dort auch Butene C4 und höhere Olefine C8+ gebildet. Im Übrigen ist es auch vorstellbar, dass in der zweiten Synthese Octen durch Tetramerisierung von Ethen entsteht.

Die zweite Synthese 2 wird so gefahren, dass die Menge an Hexen, die in der ersten Synthese 1 gebildet wird, in der zweiten Synthese 2 wieder verbraucht wird. Damit wird ein Aufbau von Hexen in der Anlage vermieden. Für den Fall, dass in der zweiten Synthese nicht genug Hexen in Octen umgesetzt werden kann, als sich in der ersten Synthese bildet, muss aus dem System Hexen ausgeschleust werden, damit die Anlage damit nicht vollläuft. Das Ausschleusen kann beispielsweise vom Kopf der dritten Kolonne 7 erfolgen. Dies ist aber nicht angestrebt und deswegen auch nicht in Figur 1 dargestellt.

Dass mehr Hexen zu C8 und C8+ umgesetzt als neu gebildet wird, was zu Problemen bei der Aufrechterhaltung des Lösemittelkreislaufs führen würde, kann durch Zusatz eines inerten Lösemittels wie Hexan vermieden werden. Das inerte Lösemittel wird anders als das Hexen nicht im Prozess gebildet, sondern muss vor dem Anfahren von außen eingefüllt werden. Falls es altert, sich also nicht ideal inert verhält, muss es von Zeit zu Zeit ausgetauscht werden.

Das aus der zweiten Synthese abgezogene zweite Reaktionsgemisch C2, C4, C6, C8, C8+, SOLV umfasst dieselben Olefine wie das erste Reaktionsgemisch, jedoch in anderer Zusammensetzung. Das inerte Lösemittel Hexan SOLV ist ebenfalls enthalten. Die zweite Synthese 2 bildet schwerpunktmäßig Octen, sodass der $C_8$-Gehalt des zweiten Reaktionsgemisches höher ist als im ersten Reaktionsgemisch. Letzteres hat wiederum einen höheren $C_4$-Gehalt.

Aufgrund der qualitativ gleichen Zusammensetzung kann das zweite Reaktionsgemisch gemeinsam mit dem ersten Reaktionsgemisch aufgearbeitet werden. Es kann unmittelbar mit in die Kolonnenserie 5, 6, 7 hereingefahren werden.

Allerdings macht es Sinn, das zweite Reaktionsgemisch zuvor erst grob destillativ aufzutrennen in eine Fraktion aus Leicht- und Mittelsiedern C2, C4, C6 und eine Schwersiederfraktion C8, C8+, wofür eine vierte Kolonne 9 vorgesehen ist. Da die zweite Synthese 2 mehr Octen und höhere Olefine macht, ist die Schwersiederfraktion C8, C8+ deutlich größer als die Leicht- und Mittelsiederfraktion C2, C4, C6. Daher kann die vierte Kolonne 9 mit vergleichsweise wenig Energieaufwand betrieben werden. Da die Schwersieder C8, C8+ der zweiten Synthese nicht durch die Kolonnenserie 5, 6, 7 hindurchgeschleust werden müssen, brauchen die drei Kolonnen 5, 6, 7 auch nicht so groß dimensioniert zu werden.

Die Vereinigung der von der vierten Kolonne stammenden Leicht- und Mittelsiederfraktion C2, C4, C6 mit dem ersten Reaktionsgemisch C2, C4, C6, C8, C8+, SOLV erfolgt in einem dritten Mischer 10, der vor der ersten Kolonne 5 angeordnet ist.

Die Schwersiederfraktion C8, C8+ aus der vierten Kolonne 9 entspricht qualitativ dem Sumpf der dritten Kolonne 7. Deshalb können beide Ströme in einem vierten Mischer 11 vereinigt und gemeinsam in einer fünften Kolonne 12 destillativ getrennt werden. Vom Kopf der fünften Kolonne wird Octen C8 als zweites Zielprodukt abgezogen, im Sumpf verbleiben die höheren Olefine C8+, die als unvermeidliches Nebenprodukt separat verwertet werden.

Nun zurück zu dem ersten Zielprodukt Buten C4, welches am Kopf der zweiten Kolonne 6 als Leichtsiederfraktion anfällt.

Das dort erhaltene Buten C4 ist nicht isomerenrein, sondern stellt vielmehr ein Isomerengemisch 1B, 2B aus 1-Buten und cis-2-Buten und trans-2-Buten dar. Mithin handelt es sich bei dem Kopfprodukt der zweiten Kolonne um lineares n-Buten. Erfreulich ist, dass es kein verzweigtes Isobuten enthält, da dieses in der ersten Synthese nicht gebildet wird. Eine aufwändige Isobuten-Abtrennung, die bei der Gewinnung von n-Buten aus $C_4$-Strömen erforderlich ist, kann damit bei diesem auf Ethen basierenden Prozess entfallen.

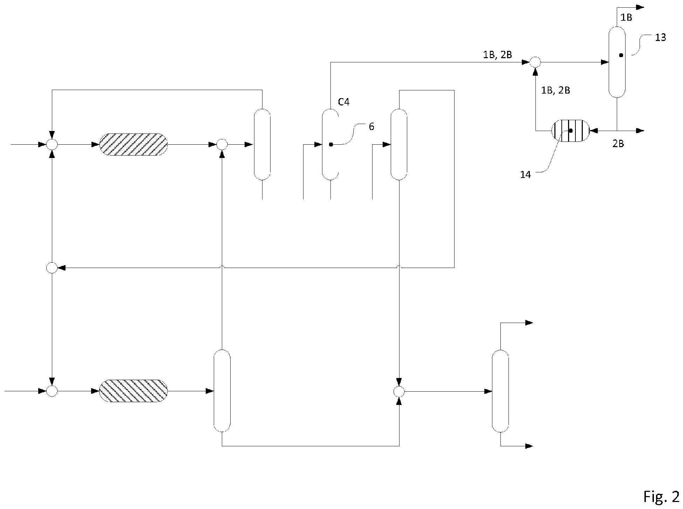

Die Wirtschaftlichkeit des Prozesses kann gesteigert werden, indem das Butengemisch C4 vom Kopf der zweiten Kolonne 6 in Richtung 1-Buten weiter aufgearbeitet wird. Dafür machen die Figuren 2, 3 und 4 jeweils einen Vorschlag.

Gemeinsames Merkmal dieser drei Varianten ist eine sechste Kolonne 13, die zur destillativen Trennung von 1-Buten 1B und 2-Buten 2B bestimmt ist. 1-Buten 1B hat einen niedrigeren Siedepunkt als cis-2-Buten und trans-2-Buten und kann daher hochrein vom Kopf der sechsten Kolonne 13 abgezogen werden.

Für die Verwendung des 2-Butens 2B am Sumpf der sechsten Kolonne 13 gibt es nun drei Möglichkeiten:

In der ersten Variante gemäß Figur 2 wird das 2-Buten einer Isomerisierung 14 unterworfen, die das 2-Buten teilweise in 1-Buten umsetzt. Nach der Isomerisierung 14 liegt wieder ein Isomerengemisch 1B, 2B aus 1-Buten und 2-Buten vor, das mit dem Kopfprodukt der zweiten Kolonne 6 vermischt und der sechsten Kolonne 13 wieder zugeführt wird. Aus thermodynamischen Gründen kann die Isomerisierung des 2-Butens nie vollständig sein. Deswegen ist es erforderlich, stetig 2-Buten 2B vom Sumpf der sechsten Kolonne 13 auszuschleusen.

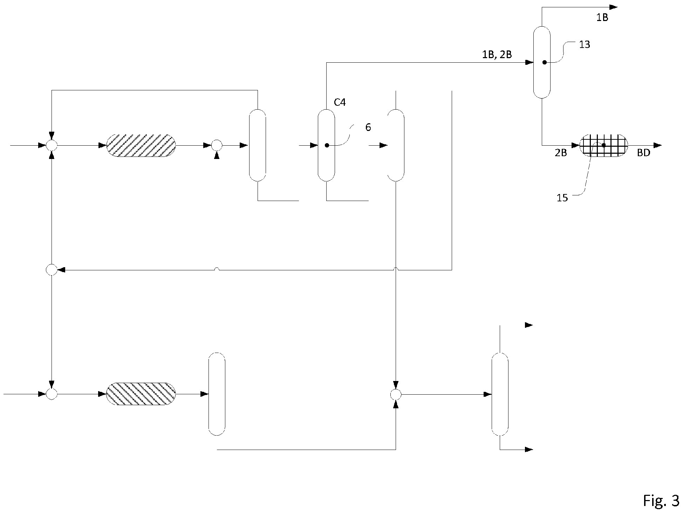

Alternativ kann das 2-Buten 2B vom Sumpf der sechsten Kolonne 13 einer oxidativen Dehydrierung 15 unterworfen werden. Dies ist in Figur 3 gezeigt. In der oxidativen Dehydrierung 15 wird das 2-Buten zu 1,3-Butadien BD umgesetzt, eine Chemikalie mit größerer Wertschöpfung als 2-Buten. Der oxidativen Dehydrierung kann auch eine Isomerisierung von 2-Buten nach 1-Buten vorangehen, da 1-Buten schneller zu Butadien reagiert als 2-Buten. Die fakultative Isomerisierung ist in Figur 3 nicht dargestellt.

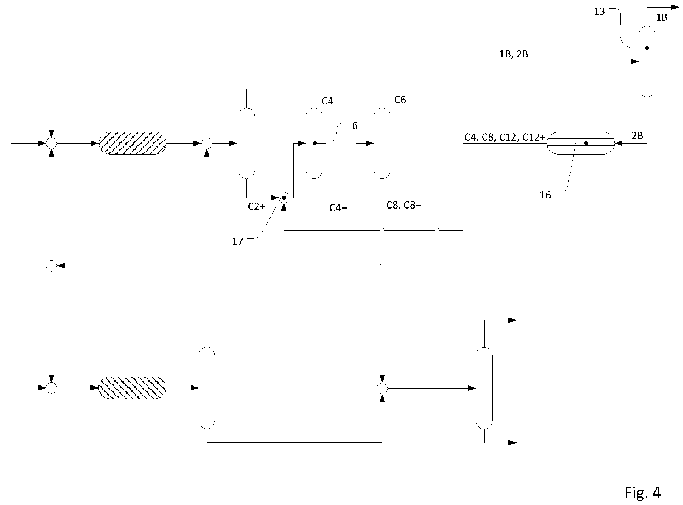

Schließlich kann gemäß Figur 4 das 2-Buten 2B vom Sumpf der sechsten Kolonne 13 einer dritten Synthese 16 zugeführt werden, in der es zumindest teilweise zu Octen oligomerisiert wird. Dies geschieht bevorzugt in einem OCTOL®-Prozess, der neben dem Dibuten noch Olefine mit zwölf und mehr Kohlenstoffatomen C12, C12+ bildet. Das auf diese Weise erhaltene dritte Reaktionsgemisch C4, C8, C12, C12+ wird mit dem Sumpf C2+ der ersten Kolonne 5 in einem sechsten Mischer 17 vermischt und der zweiten Kolonne 6 zugeführt. Mithin erfolgt die Aufarbeitung des dritten Reaktionsgemisches gemeinsam mit dem ersten und dem zweiten Reaktionsgemisch.

Den drei in den Figuren 2, 3 und 4 gezeigten Varianten zur Aufarbeitung der Buten- haltigen Leichtsiederfraktion C4 ist gemein, dass diese nicht mit dem Lösemittel SOLV befrachtet wird, da dieses ja höher siedet als die Butene und daher im Sumpf der Kolonne 6 verbleibt, von deren Kopf die Butene als Leichtsiederfraktion C4 abgetrennt werden. Damit wird einerseits der Energiebedarf für die Kolonne 6 gesenkt und andererseits die C4-Aufarbeitung nicht mit inertem Material beaufschlagt, sodass die Apparate kleiner ausfallen können.

Die Erfindung soll nun anhand von Beispielen näher erläutert werden.

Beispiel 1

Oligomerisierung von Ethen in n-Hexan mit Fokus auf 1-Buten (erste Reaktion)

15.5 g eines heterogenen Katalysators basierend auf Nickel und Silica-Alumina (vgl. US 2581228) wurden in einen außen mit Öl temperierten Rohrreaktor von 1 m Länge und 6 mm Innendurchmesser gefüllt. Anschließend wurde ein Gemisch aus 17 Massen-% Ethen, 77 Massen-% n-Hexan und 6 Massen-% des internen Standards n-Heptan mit einem Gesamtmengenstrom von 100 g/h bei einer Temperatur von 70 °C gefahren (WHSV = 6.4/h). Der Druck wurde auf 30 bar konstant gehalten. Nach einer Zeit von 60 h war ein Zustand erreicht, in dem sich der Umsatz nicht mehr änderte. Die Ergebnisse sind in Tabelle 1 zusammengefasst. Zur weiteren Analyse wurde die Produktfraktion in einen hydrierenden Gaschromatographen gespritzt. Die Zusammensetzungen der hydrierten C8-Fraktion sind ebenfalls in Tabelle 1 zusammengefasst.

**Beispiel 2**

Oligomerisierung von Ethen in n-Hexen mit Fokus auf 1-Buten (erste Reaktion)

Analog zu Beispiel 1 wurden 15.5 g des gleichen Katalysators in einen außen mit Öl temperierten Rohrreaktor von 1 m Länge und 6 mm Innendurchmesser gefüllt. Anschließend wurde ein Gemisch aus 20 Massen-% Ethen, 73 Massen-% n-Hexen und 7 Massen-% des internen Standards n-Heptan mit einem Gesamtmengenstrom von 105 g/h bei einer Temperatur von 70 °C gefahren (WHSV = 6.8/h). Der Druck wurde auf 30 bar konstant gehalten. Nach einer Zeit von 73 h war ein Zustand erreicht, in dem sich der Umsatz nicht mehr änderte. Die Ergebnisse sowie die Zusammensetzung der hydrierten C8-Fraktion sind in Tabelle 1 zusammengefasst.

**Beispiel 3**

Oligomerisierung von Ethen in einem n-Hexen/n-Hexan-Gemisch mit Fokus auf 1-Buten (erste Reaktion)

Analog zu Beispiel 1 wurden 4.1 g des gleichen Katalysators in einen außen mit Öl temperierten Rohrreaktor von 1 m Länge und 6 mm Innendurchmesser gefüllt. Anschließend wurde ein Gemisch aus 17 Massen-% Ethen, 45 Massen-% n-Hexen und 38 Massen-% n-Hexan mit einem Gesamtmengenstrom von 100 g/h bei einer Temperatur von 70 °C gefahren (WHSV = 24.2/h). Der Druck wurde auf 30 bar konstant gehalten. Nach einer Zeit von 72 h war ein Zustand erreicht, in dem sich der Umsatz nicht mehr änderte. Die Ergebnisse sowie die Zusammensetzungen der hydrierten $C_8$-Fraktion sind in Tabelle 1 zusammengefasst.

**Beispiel 4**

Oligomerisierung von Ethen in einem n-Hexen/n-Hexan-Gemisch mit Fokus auf Octen (zweite Reaktion)

Analog zu Beispiel 3 wurden 15.5 g eines Katalysators mit einem Gemisch aus 5 Massen-% Ethen, 53 Massen-% n-Hexen, 30 Massen-% n-Hexan und 12 Massen-% des internen Standards n-Heptan mit einem Gesamtmengenstrom von 100 g/h bei einer Temperatur von 70 °C gefahren (WHSV = 6.4/h). Der Druck wurde auf 30 bar konstant gehalten. Nach einer Zeit von 72 h war ein Zustand erreicht, in dem sich der Umsatz nicht mehr änderte. Die Ergebnisse sowie die Zusammensetzungen der hydrierten C8-Fraktion sind in Tabelle 1 zusammengefasst.

Tabelle 1: Ergebnisse der Beispiele 1 bis 4

| Beispiel | Umsatz | C4 | C6 | C8 | C8+ | 1-Buten | 2-Buten | Sel. nO | Sel. MH | Sel. DMH |
|---|---|---|---|---|---|---|---|---|---|---|
| 1 | 99% | 68% | 21% | 8% | 3% | 19% | 81% | 41% | 51% | 7%[a] |
| 2 | 98% | 58% | 8%[e] | 25% | 9% | 45% | 55% | 30% | 68% | 2%[b] |
| 3 | 92% | 71% | -2%[e] | 23% | 8% | 33% | 67% | 30% | 68% | 2%[c] |
| 4 | 99% | 51% | 15%[e] | 29% | 7% | 31% | 69% | 31% | 66% | 1%[d] |

In Tabelle 1 bedeuten die Fußnoten: a - d) Der Isoindex des C8-Gemisches beträgt für a) 0.65, für b) und c) 0.72 und für d) 0.68; e) C6 resultiert aus neu gebildetem Hexen abzüglich des verbrauchten Hexens.
Die Abkürzung Sel. steht für Selektivität. nO steht für n-Octen, MH für Methylhexen und DMH für Dimetyhlhexen.

**Fazit**

Die Reaktion in purem n-Hexan (Beispiel 1) resultiert in einer hohen C4- und einer niedrigen C8-Selektivität. Gleichzeitig wird das 1-Buten unter diesen Bedingungen verstärkt isomerisiert bzw. weiter umgesetzt, so dass die 1-Buten-Selektivität vergleichsweise gering ausfällt. Zudem fällt auf, dass recht viel C6 gebildet wird. Dieses kann durch Reaktionsbedingungen in purem n-Hexan (Beispiel 2) durch Weiterreaktion zu C8 größtenteils verwertet werden, wobei jedoch gleichzeitig die C4-Selektivität sinkt und der C8+-Anteil durch Oligomerisierung des C6 ansteigt.
Durch Verdünnen mittels n-Hexan kann die Reaktivität des n-Hexens herabgesetzt werden, so dass der Verbrauch des C6 gezielter gesteuert werden und damit der Kreislauf besser aufrechterhalten werden kann. Die Bildung von C8 und C8+ fällt hierbei etwas geringer aus. Zudem lässt sich durch Herabsetzen der Verweilzeit (WHSV 24 statt 6) die C4- und die 1-Buten-Selektivität anheben (Beispiel 3). Bei gleichen Hexen/Hexan-Verhältnissen führt ein geringerer Ethen-Gehalt (Beispiel 4 im Vergleich zu Beispiel 3) zu einer verminderten Bildung von C4 (51% vs. 71%) und einer verstärkten Bildung von C8 (29% vs. 23%), ganz im Sinne der Synthese 2.

In allen Fällen weist das C8-Gemisch einen deutlich günstigeren Isoindex auf als auf dem Wege der Buten-Oligomerisierung derzeit erzielbar ist (0.65-0.72 vs. > 0.9).

In Summe wird im Falle einer Verdünnung des n-Hexens mit n-Hexan bei diesen Reaktionsbedingungen in Synthese 1 (Beispiel 3) etwas mehr Hexen verbraucht als neu gebildet, während in Synthese 2 (Beispiel 4) mehr Hexen gebildet als verbraucht wird; in der Gesamtbilanz ergibt sich damit in diesem Fall ein gewisser purge-C6-Strom, der aus dem Kreislauf entfernt werden muss. Dabei wird in Synthese 1 (Beispiel 3) wie gewünscht bevorzugt 1-Buten gebildet, während in Synthese 2 (Beispiel 4) vergleichsweise mehr C8 produziert wird.

Abschließend sollen die wesentlichen Aspekte und Vorteile der Erfindung zusammengefasst werden:

Das hier vorgestellte, heterogen katalysierte Verfahren dient der kombinierten Herstellung von Buten und Octen aus Ethen. Es umfasst die simultane Durchführung zweier Reaktionen, nämlich eine erste Synthese vornehmlich von $C_2$ zu $C_4$ und eine zweite Synthese von $C_2$ und $C_6$ zu $C_8$. Beide Reaktionen werden räumlich voneinander getrennt betrieben. Beide Synthesen werden in Gegenwart eines inerten Lösemittels durchgeführt, dessen Siedelage zwischen den Butenen und den Octenen liegt. In der ersten Synthese wird neben dem ersten Zielprodukt Buten auch noch Hexen und Octen gebildet. Das in der ersten Synthese im Zuge der $C_2$-Trimerisierung gebildete Hexen wird als Edukt für die zweite Synthese verwendet. Dort wird es mit Ethen zu Octen umgesetzt, dem zweiten Zielprodukt. Das eigentlich unerwünschte Nebenprodukt der ersten Synthese Hexen wird somit zur Bildung von Octen weiterverwertet. Schließlich wird das Lösemittel laufend im Kreis gefahren. Dies ermöglicht es, beide Synthesen in der Flüssigphase durchzuführen und die Selektivität der Reaktionen zu steuern. Da die Siedelage des Lösemittels im Wesentlichen dem des Nebenprodukts Hexen entspricht, kann das Nebenprodukt gemeinsam mit dem Lösemittel als Mittelsieder aus dem ersten Reaktionsgemisch abgetrennt und in die zweite Reaktion überführt werden.

**Bezugzeichenliste**

| | |
|---|---|
| 1 | erste Synthese |
| 2 | zweite Synthese |
| 3 | erster Mischer |
| 4 | zweiter Mischer |
| 5 | erste Kolonne |
| 6 | zweite Kolonne |
| 7 | dritte Kolonne |
| 8 | Teiler |
| 9 | vierte Kolonne |
| 10 | dritter Mischer |
| 11 | vierter Mischer |
| 12 | fünfte Kolonne |
| 13 | sechste Kolonne |
| 14 | Isomerisierung |
| 15 | oxidative Dehydrierung |
| 16 | dritte Synthese |
| 17 | sechster Mischer |
| C2 | Ethen |
| C4 | Buten (Leichtsiederfraktion, Kopf der zweiten Kolonne) |
| C6 | Hexen |
| C8 | Octen |
| C8+ | höhere Olefine |
| C2, C6, SOLV | erstes bzw. zweites Einsatzgemisch |
| C2, C4, C6, C8, C8+, SOLV | erster bzw. zweiter Rektionsaustrag |
| C2+ | Sumpf der ersten Kolonne |
| C4+ | Sumpf der zweiten Kolonne |
| C6, SOLV | Mittelsiederfraktion bzw. Kopf der dritten Kolonne |
| C8, C8+ | Schwersiederfraktion bzw. Sumpf der dritten Kolonne |
| C2, C4, C6, SOLV | Leicht- und Mittelsiederfraktion |
| 1B | 1-Buten |
| 2B | 2-Buten (cis und trans) |

| 1B, 2B | Isomerengemisch |
|---|---|
| BD | Butadien |
| C4, C8, C12, C12+ | drittes Reaktionsgemisch |
| SOLV | Lösemittel (Hexan) |

**Patentansprüche**

1.  Verfahren zur kombinierten Herstellung von Buten und Octen aus Ethen mit den folgenden Schritten:

    a) Bereitstellen eines Lösemittels, dessen Siedepunkt bzw. Siedebereich oberhalb der Siedepunkte der Butene und unterhalb der Siedepunkte der Octene liegt und bei welchem es sich um ein inertes Lösemittel handelt oder um Hexen allein oder um Hexen gemischt mit Pentan oder Hexan oder Heptan oder um eine Mischung aus Pentan, Hexan, Heptan;
    b) Bereitstellen eines ersten Einsatzgemisches enthaltend zumindest das Lösemittel und darin gelöstes Ethen;
    c) Bereitstellen eines zweiten Einsatzgemisches enthaltend zumindest Hexen, das Lösemittel, sowie im Lösemittel und/oder im Hexen gelöstes Ethen;
    d) Überführen des ersten Einsatzgemisches in eine erste Synthese und des zweiten Einsatzgemisches in eine zweite Synthese, wobei erste und zweite Synthese räumlich voneinander getrennt sind;
    e) in der ersten Synthese Oligomerisieren zumindest eines Teils des im ersten Einsatzgemisch enthaltenden Ethens in Gegenwart eines ersten heterogenen Katalysators und in Gegenwart des Lösemittels unter Erhalt eines ersten Reaktionsgemisches umfassend zumindest das Lösemittel, Buten und Hexen;
    f) Abtrennen einer Buten enthaltenden Leichtsiederfraktion aus dem ersten Reaktionsgemisch bzw. aus einem auf dem ersten Reaktionsgemisch basierenden Stoffstrom;
    g) Abtrennen einer Hexen und das Lösemittel enthaltenden Mittelsiederfraktion aus dem ersten Reaktionsgemisch bzw. aus einem auf dem ersten Reaktionsgemisch basierenden Stoffstrom;
    h) Verwenden zumindest eines Teils der Mittelsiederfraktion im Zuge der Bereitstellung des zweiten Einsatzgemischs;
    i) in der zweiten Synthese Umsetzen zumindest eines Teils des im zweiten Einsatzgemisch enthaltenden Ethens mit zumindest einem Teil des im zweiten Einsatzgemisch enthaltenden Hexens in Gegenwart eines zweiten heterogenen Katalysators und in Gegenwart des Lösemittels unter Erhalt eines zweiten Reaktionsgemisches umfassend zumindest Octen und das Lösemittel.

2.  Verfahren nach Anspruch 1, bei welchem im ersten Reaktionsgemisch und/oder im zweiten Reaktionsgemisch enthaltendes Ethen abgetrennt und zur Bereitstellung des ersten und/oder zweiten Einsatzgemisches verwendet wird.

3.  Verfahren nach Anspruch 1 oder 2, bei welchem im zweiten Reaktionsgemisch enthaltendes Buten gemeinsam mit dem im ersten Reaktionsgemisch enthaltenden Buten aufgearbeitet wird.

4.  Verfahren nach einem der vorhergehenden Ansprüche, bei welchem im zweiten Reaktionsgemisch enthaltendes Hexen abgetrennt und zumindest teilweise in die zweite Synthese zurückgeführt wird.

5.  Verfahren nach einem der vorhergehenden Ansprüche, **dadurch gekennzeichnet, dass** es sich bei dem Lösemittel um einen Kohlenwasserstoff mit fünf oder sechs oder sieben Kohlenstoffatomen handelt oder um ein Gemisch mehrerer solcher Kohlenwasserstoffe, insbesondere, dass es sich bei dem Lösemittel um n-Hexan handelt.

6.  Verfahren nach einem der vorhergehenden Ansprüche, **dadurch gekennzeichnet, dass** der Anteil des Ethens an dem ersten Einsatzgemisch zwischen 1 und 50 Gew.-%, beträgt, und dass die erste Synthese bei einer Temperatur zwischen 20 °C und 150 °C und bei einem Druck zwischen $1*10^5$ Pa und $50*10^5$ Pa erfolgt, wobei der Anteil des Ethens an dem ersten Einsatzgemisch und die Reaktionsbedingungen der ersten Synthese so gewählt sind, dass das Lösemittel in der flüssigen Phase vorliegt.

7.  Verfahren nach Anspruch 6, **dadurch gekennzeichnet, dass** der Anteil des Ethens an dem ersten Einsatzgemisch und die Reaktionsbedingungen der ersten Synthese so gewählt sind, dass in der ersten Synthese das Ethen vollständig in den in der flüssigen Phase vorliegenden Lösemittel gelöst ist.

8.  Verfahren nach Anspruch 6, **dadurch gekennzeichnet, dass** der Anteil des Ethens an dem ersten Einsatzgemisch und die Reaktionsbedingungen der ersten Synthese so gewählt sind, dass in der ersten Synthese das Ethen teilweise in einer Gasphase vorliegt und teilweise in dem in der flüssigen Phase vorliegenden Lösemittel gelöst ist.

9. Verfahren nach einem der vorhergehenden Ansprüche, **dadurch gekennzeichnet, dass** der Anteil des Ethens an dem zweiten Einsatzgemisch zwischen 0,1 und 30 Gew.-%, beträgt, und dass die zweite Synthese bei einer Temperatur zwischen 20 und 150 °C und bei einem Druck zwischen $1*10^5$ Pa und $50*10^5$ Pa erfolgt, wobei der Anteil des Ethens an dem zweiten Einsatzgemisch und die Reaktionsbedingungen der zweiten Synthese so gewählt sind, dass das Hexen und das Lösemittel in der flüssigen Phase vorliegen und das Ethen darin vollständig gelöst ist.

10. Verfahren nach einem der vorhergehenden Ansprüche, **dadurch gekennzeichnet, dass** das zweite Einsatzgemisch mehr Gewichtsanteil Hexen als Ethen enthält.

11. Verfahren nach Anspruch 10, **dadurch gekennzeichnet, dass** der Anteil des Ethens an dem zweiten Einsatzgemisch weniger als 30 Gew.-%, ganz besonders bevorzugt weniger als 20 Gew.-% beträgt.

12. Verfahren nach einem der vorhergehenden Ansprüche, **dadurch gekennzeichnet, dass** als erster und/oder zweiter heterogener Katalysator ein Festkörper eingesetzt wird, welcher mindestens zwei Komponenten enthält, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus $Al_2O_3$, $SiO_2$, $TiO_2$, $ZrO_2$ ausgewähltes Metalloxid umfasst.

## Claims

1. Process for the combined preparation of butene and octene from ethene, which comprises the following steps:

    a) provision of a solvent whose boiling point or boiling range is above the boiling points of the butenes and below the boiling points of the octenes and which solvent is an inert solvent or is hexene alone or is hexene admixed with pentane or hexane or heptane or is a mixture of pentane, hexane, heptane;
    b) provision of a first feed mixture containing at least the solvent and ethene dissolved therein;
    c) provision of a second feed mixture containing at least hexene, the solvent and also ethene dissolved in the solvent and/or in the hexene;
    d) transfer of the first feed mixture into a first synthesis and of the second feed mixture into a second synthesis, where the first and second syntheses are physically separate from one another;
    e) oligomerisation of at least part of the ethene present in the first feed mixture in the presence of a first heterogeneous catalyst and in the presence of the solvent in the first synthesis to give a first reaction mixture comprising at least the solvent, butene and hexene;
    f) separation of a butene-containing low boiler fraction from the first reaction mixture or from a stream based on the first reaction mixture;
    g) separation of an intermediate boiler fraction containing hexene and the solvent from the first reaction mixture or from a stream based on the first reaction mixture;
    h) use of at least part of the intermediate boiler fraction in the course of the provision of the second feed mixture;
    i) reaction of at least part of the ethene present in the second feed mixture with at least part of the hexene present in the second feed mixture in the presence of a second heterogeneous catalyst and in the presence of the solvent in the second synthesis to give a second reaction mixture comprising at least octene and the solvent.

2. Process according to Claim 1, wherein ethene present in the first reaction mixture and/or in the second reaction mixture is separated off and used for provision of the first and/or second feed mixture.

3. Process according to Claim 1 or 2, wherein butene present in the second reaction mixture is worked up together with the butene present in the first reaction mixture.

4. Process according to any of the preceding claims, wherein hexene present in the second reaction mixture is separated off and at least partly recirculated to the second synthesis.

5. Process according to any of the preceding claims, **characterized in that** the solvent is a hydrocarbon having five or six or seven carbon atoms or a mixture of a plurality of such hydrocarbons, in particular **in that** the solvent is n-hexane.

6. Process according to any of the preceding claims, **characterized in that** the proportion of ethene in the first feed mixture is in the range from 1 to 50% by weight and **in that** the first synthesis is carried out at a temperature in the range from 20°C to 150°C and at a pressure in the range from $1*10^5$ Pa to $50*10^5$ Pa, where the proportion of ethene in the first feed mixture and the reaction conditions of the first synthesis are selected so that the solvent is present in the liquid phase.

7. Process according to Claim 6, **characterized in that** the proportion of ethene in the first feed mixture and the reaction conditions of the first synthesis are selected so that the ethene is completely dissolved in the solvents present in the liquid phase in the first synthesis.

8. Process according to Claim 6, **characterized in that** the proportion of ethene in the first feed mixture and the reaction conditions of the first synthesis are selected so that the ethene is partly present in a gas phase and is partly dissolved in the solvent present in the liquid phase in the first synthesis.

9. Process according to any of the preceding claims, **characterized in that** the proportion of ethene in the second feed mixture is in the range from 0.1 to 30% by weight and **in that** the second synthesis is carried out at a temperature in the range from 20 to 150°C and at a pressure in the range from $1*10^5$ Pa to $50*10^5$ Pa, where the proportion of ethene in the second feed mixture and the reaction conditions of the second synthesis are selected so that the hexene and the solvent are present in the liquid phase and the ethene is completely dissolved therein.

10. Process according to any of the preceding claims, **characterized in that** the second feed mixture contains a greater proportion by weight of hexene than of ethene.

11. Process according to Claim 10, **characterized in that** the proportion of ethene in the second feed mixture is less than 30% by weight, very particularly preferably less than 20% by weight.

12. Process according to any of the preceding claims, **characterized in that** a solid which contains at least two components, where the first component comprises at least one element selected from among Ni, Cr, Fe, Ti which is present in metallic and/or oxidic and/or hydridic form and the second component comprises at least one metal oxide selected from among $Al_2O_3$, $SiO_2$, $TiO_2$, $ZrO_2$, is used as first and/or second heterogeneous catalyst.

**Revendications**

1. Procédé de fabrication combinée de butène et d'octène à partir d'éthène, comprenant les étapes suivantes :

a) la préparation d'un solvant, dont le point d'ébullition ou la plage d'ébullition se situe au-dessus des points d'ébullition des butènes et en dessous des points d'ébullition des octènes, et qui est un solvant inerte ou l'hexène seul ou l'hexène mélangé avec du pentane ou de l'hexane ou de l'heptane ou un mélange de pentane, d'hexane, d'heptane ;
b) la préparation d'un premier mélange de départ contenant au moins le solvant et l'éthène dissous dans celui-ci ;
c) la préparation d'un deuxième mélange de départ contenant au moins de l'hexène, le solvant, ainsi que de l'éthène dissous dans le solvant et/ou dans l'hexène ;
d) le transfert du premier mélange de départ dans une première synthèse et du deuxième mélange de départ dans une deuxième synthèse, la première et la deuxième synthèse étant séparées l'une de l'autre dans l'espace ;
e) dans la première synthèse, l'oligomérisation d'au moins une partie de l'éthène contenu dans le premier mélange de départ en présence d'un premier catalyseur hétérogène et en présence du solvant pour obtenir un premier mélange réactionnel comprenant au moins le solvant, du butène et de l'hexène ;
f) la séparation d'une fraction de composants de point d'ébullition faible contenant du butène du premier mélange réactionnel ou d'un courant de matière à base du premier mélange réactionnel ;
g) la séparation d'une fraction de composants de point d'ébullition moyen contenant de l'hexène et le solvant du premier mélange réactionnel ou d'un courant de matière à base du premier mélange réactionnel ;
h) l'utilisation d'au moins une partie de la fraction de composants de point d'ébullition moyen au cours de la préparation du deuxième mélange de départ ;
i) dans la deuxième synthèse, la mise en réaction d'au moins une partie de l'éthène contenu dans le deuxième mélange de départ avec au moins une partie de l'hexène contenu dans le deuxième mélange de départ en présence d'un deuxième catalyseur hétérogène et en présence du solvant pour obtenir un deuxième mélange réactionnel comprenant au moins de l'octène et le solvant.

**2.** Procédé selon la revendication 1, dans lequel l'éthène contenu dans le premier mélange réactionnel et/ou dans le deuxième mélange réactionnel est séparé et utilisé pour la préparation du premier et/ou du deuxième mélange de départ.

**3.** Procédé selon la revendication 1 ou 2, dans lequel le butène contenu dans le deuxième mélange réactionnel est traité conjointement avec le butène contenu dans le premier mélange réactionnel.

**4.** Procédé selon l'une quelconque des revendications précédentes, dans lequel l'hexène contenu dans le deuxième mélange réactionnel est séparé et recyclé au moins en partie dans la deuxième synthèse.

**5.** Procédé selon l'une quelconque des revendications précédentes, **caractérisé en ce que** le solvant est un hydrocarbure contenant cinq ou six ou sept atomes de carbone ou un mélange de plusieurs tels hydrocarbures, le solvant étant notamment le n-hexane.

**6.** Procédé selon l'une quelconque des revendications précédentes, **caractérisé en ce que** la proportion d'éthène dans le premier mélange de départ est comprise entre 1 et 50 % en poids, et **en ce que** la première synthèse a lieu à une température comprise entre 20 °C et 150 °C, et à une pression comprise entre $1*10^5$ Pa et $50*10^5$ Pa, la proportion d'éthène dans le premier mélange de départ et les conditions de réaction de la première synthèse étant choisies de sorte que le solvant se présente dans la phase liquide.

**7.** Procédé selon la revendication 6, **caractérisé en ce que** la proportion d'éthène dans le premier mélange de départ et les conditions de réaction de la première synthèse sont choisies de sorte que l'éthène dans la première synthèse soit entièrement dissous dans le solvant présent dans la phase liquide.

**8.** Procédé selon la revendication 6, **caractérisé en ce que** la proportion d'éthène dans le premier mélange de départ et les conditions de réaction de la première synthèse sont choisies de sorte que l'éthène dans la première synthèse se présente partiellement dans une phase gazeuse et soit partiellement dissous dans le solvant présent dans la phase liquide.

**9.** Procédé selon l'une quelconque des revendications précédentes, **caractérisé en ce que** la proportion d'éthène dans le deuxième mélange de départ est comprise entre 0,1 et 30 % en poids, et **en ce que** la deuxième synthèse a lieu à une température comprise entre 20 et 150 °C et à une pression comprise entre $1*10^5$ Pa et $50*10^5$ Pa, la proportion d'éthène dans le deuxième mélange de départ et les conditions de réaction de la deuxième synthèse étant choisies de sorte que l'hexène et le solvant soient présents dans la phase liquide et l'éthène soit entièrement dissous dans ceux-ci.

**10.** Procédé selon l'une quelconque des revendications précédentes, **caractérisé en ce que** le deuxième mélange de départ contient une proportion en poids d'hexène supérieure à celle d'éthène.

**11.** Procédé selon la revendication 10, **caractérisé en ce que** la proportion de l'éthène dans le deuxième mélange de départ est inférieure à 30 % en poids, de manière tout particulièrement préférée inférieure à 20 % en poids.

**12.** Procédé selon l'une quelconque des revendications précédentes, **caractérisé en ce qu'**un corps solide est utilisé en tant que premier et/ou deuxième catalyseur hétérogène, qui contient au moins deux composants, le premier composant comprenant au moins un élément choisi parmi Ni, Cr, Fe, Ti, qui se présente sous forme métallique et/ou oxydique et/ou hydrurique, et le deuxième composant comprenant au moins un oxyde métallique choisi parmi $Al_2O_3$, $SiO_2$, $TiO_2$, $ZrO_2$.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008007081 A1
- EP 1029839 A1
- EP 2582648 B1
- WO 2005123884 A
- WO 2005123633 A
- US 20130066128 A1
- US 8637722 B2
- WO 2010117539 A1
- US 20130158321 A1
- EP 0319208 A1
- EP 0718036 A1
- DE 102013226370
- US 2581228 A

**In der Beschreibung aufgeführte Nicht-Patentliteratur**

- **F. GEILEN ; G. STOCHNIOL ; S. PEITZ ; E. SCHULTE-KOERNE.** *Ullmann's Encyclopedia of Industrial Chemistry,* 2013
- The HÜLS OCTOL Process: Heterogeneously catalyzed dimerization of n-butenes and other olefins. **B. SCHOLZ.** DGMK-Tagung. April 1989, 21, , 22
- **R.H. FRIEDLANDER ; D.J. WARD ; F. OBENAUS ; F. NIERLICH ; J. NEUMEISTER.** Make plasticizer olefins via n-butene dimerization. *Hydrocarbon Processing,* Februar 1986, 31-33
- **F. NIERLICH.** Oligomerize for better gasoline. *Hydrocarbon Processing,* Februar 1992, 45-46